# FASCICULE DE BREVET EUROPEEN

(11) **EP 0 805 665 B1**
(45) Date de publication et mention de la délivrance du brevet: **22.03.2000**
(21) Numéro de dépôt: 96901401.8
(22) Date de dépôt: 17.01.1996
(51) Int. Cl.: A61F 13/15

(54) **ARTICLE D'HYGIENE ABSORBANT JETABLE AVEC BARRIERES D'ETANCHEITE LONGITUDINALES EXTENSIBLES FORMANT POCHE**
LONGITUDINAL AUFGEBRACHTE, UND EINE AUFFANGTASCHE FORMENDE DEHNBARE DICHTUNGSWAND FÜR WEGWERFWINDEL
DISPOSABLE ABSORBENT SANITARY ARTICLE WITH STRETCHABLE LONGITUDINAL SEALS FORMING A POUCH

(30) Priorité: 23.01.1995 FR 9500918
(43) Date de publication de la demande: 12.11.1997
(73) Titulaire: SCA HYGIENE PRODUCTS SA, 59126 Linselles (FR)
(72) Inventeur: BONJOUR, Emmanuel, F-74140 Messery (FR)
(74) Mandataire: Casalonga, Axel
(86) Numéro de dépôt international: FR9600071
(87) Numéro de publication internationale: WO9622755

(56) Documents cités:
- EP-A- 0 567 105
- FR-A- 2 711 057

## Description

L'invention concerne, d'une manière générale, un article d'hygiène absorbant jetable, tel qu'une couche-culotte absorbante jetable ouverte destinée aux enfants en bas âge ou aux personnes incontinentes, ou une culotte-couche absorbante jetable destinée à l'apprentissage de la propreté chez les enfants, du type comprenant un coussin absorbant disposé entre une feuille extérieure de support imperméable aux liquides et une feuille de couverture ou de surface intérieure perméable aux liquides.

Plus particulièrement, l'invention concerne un tel article d'hygiène absorbant jetable dont la feuille de surface intérieure comporte une ouverture longitudinale de forme allongée destinée à recevoir l'urine et les selles et au moins deux plis longitudinaux élastifiés s'étendant sur toute la longueur dudit article d'hygiène de part et d'autre de l'ouverture longitudinale, de façon à former une poche d'étanchéité et de confinement des exsudats.

Des articles d'hygiène de ce type sont connus par exemple par le brevet US-A-4662877 (Johnson & Johnson). Selon ce document, la feuille de surface intérieure de l'article d'hygiène est de nature hydrophobe et est pourvue d'une ouverture de forme allongée, décalée vers l'avant de l'article. Les bords longitudinaux de l'ouverture sont pourvus d'éléments élastiques fixés à l'état tendu.

La demande de brevet français FR-A-2 695 314 (FR-A-9210601 (Peaudouce)) décrit également une couche-culotte dont la feuille de surface intérieure comporte une ouverture allongée centrale et deux doubles plis longitudinaux élastifiés, s'étendant sur toute la longueur de la couche-culotte, situés de part et d'autre de l'ouverture centrale. Les deux replis de chaque double plis sont solidarisés l'un à l'autre sur toute la longueur des doubles plis.

La demande de brevet français FR-93 14 296 (Peaudouce), non publiée, décrit aussi une couche-culotte du même type, avec les doubles plis pouvant être rabattus vers l'extérieur dans une zone longitudinale allant du bord transversal avant de l'ouverture jusqu'au bord transversal avant de la couche-culotte, de façon à former des petites barrières longitudinales pour empêcher les fuites d'urine.

La demande de brevet européen EP-A-357 298 (The Procter and Gamble Company) décrit une couche-culotte dont la feuille de surface perméable à l'urine comporte un passage qui permet la communication des matières fécales avec le coussin absorbant, afin de les isoler de la peau de l'utilisateur. Ce passage est une ouverture oblongue légèrement décalée vers l'arrière de l'article. Des éléments élastiques sont fixés à la feuille de surface à proximité de l'ouverture pour assurer une contraction de la feuille de surface.

Les demandes de brevets européens EP-A-508 477, EP-A-563 971, EP-A-565 058 et EP-A-567 105 (Uni-Charm Corporation) décrivent également des couche-culottes dont la feuille de surface intérieure comporte une ouverture centrale de forme allongée, cette ouverture centrale pouvant être munie d'une ou de deux paires de volets longitudinaux latéraux dont les bords libres peuvent être élastifiés.

Bien que ces structures décrites dans l'art antérieur s'avèrent relativement efficaces, il serait encore souhaitable d'améliorer l'acquisition et l'isolation des exsudats corporels, tels que les selles et l'urine, et également l'étanchéité vis à vis des risques de fuites transversales d'urine. L'invention a donc pour but de fournir un article d'hygiène absorbant jetable qui présente à la fois une meilleure acquisition et stockage des exsudats et une étanchéité améliorée vis à vis des fuites transversales d'urine.

Selon l'invention, on réalise un article d'hygiène absorbant jetable, tel qu'une couche-culotte, comprenant une feuille extérieure de support imperméable aux liquides, de forme générale rectangulaire, un coussin absorbant de forme générale rectangulaire, fixé sur ladite feuille extérieure de support et d'une feuille de surface intérieure perméable aux liquides recouvrant le coussin absorbant. Cette feuille de surface intérieure est fixée sur son pourtour à la feuille extérieure de support.

L'article d'hygiène comprend, en outre, des moyens d'attache pour permettre sa fermeture autour de la taille d'un utilisateur.

L'article comprend, aussi, des éléments élastiques longitudinaux fixés à l'état tendu à la feuille de support à l'extérieur des bords longitudinaux du coussin absorbant, au moins dans une zone d'entrejambes.

La feuille de surface intérieure perméable aux liquides est pourvue d'une ouverture longitudinale, généralement de forme oblongue ou allongée centrée sur le coussin absorbant, et de largeur inférieure à celui-ci. La feuille de surface intérieure est également fixée au coussin absorbant directement ou indirectement sauf dans une zone située en dessous de l'ouverture, qui, de préférence, est de dimension supérieure à l'ouverture de façon à former une poche de confinement continue au-dessus du coussin absorbant comprenant deux volets longitudinaux d'étanchéité, se prolongeant par deux volets transversaux, dont les bords libres sont constitués par les bords de l'ouverture longitudinale.

La feuille de surface intérieure comprend également au moins deux premiers plis longitudinaux, sensiblement parallèles à l'axe longitudinal médian de l'article, s'étendant sur toute la longueur dudit article, de part et d'autre de l'ouverture longitudinale, formant des gaines tubulaires fermées, dans lesquelles est fixé, à l'état tendu, au moins un élément élastique longitudinal.

Selon l'invention la feuille de surface intérieure est pourvue d'au moins deux plis longitudinaux supplémentaires, s'étendant sur toute la longueur de l'article et disposés respectivement entre chacun des premiers plis longitudinaux et la zone longitudinale latérale de fixation correspondante de la feuille de surface intérieure au reste de l'article. Selon un mode de réalisation préférentiel, chacun de ces plis supplémentaires est solidarisé de la feuille de surface intérieure à chacune de ses extrémités longitudinales, à proximité des bords transversaux de l'article, dans une première zone de longueur réduite ; chacun de ces plis est libre de se déplier dans une seconde zone correspondant à la majeure partie de la longueur de l'article.

Du fait que ces plis supplémentaires sont libres de se déplier sur pratiquement toute leur longueur, sous l'action des premiers plis élastifiés qui soulèvent la feuille de surface le long des bords longitudinaux de l'ouverture, on obtient ainsi une augmentation substantielle de la hauteur des volets longitudinaux d'étanchéité, et donc une meilleure adaptation desdits volets longitudinaux au corps de l'utilisateur, ce qui entraîne une amélioration de l'efficacité anti-fuites de la poche de confinement, tout en conservant la même largeur de l'ouverture. Sans la présence de ces plis supplémentaires il n'y aurait pas d'autres solutions pour augmenter la hauteur des volets longitudinaux d'une telle poche de confinement, que de réduite la largeur de l'ouverture, ceci au détriment d'une adaptation optimale de l'ouverture à l'anatomie de l'utilisateur et de l'acquisition la plus efficace des selles et de l'urine par le coussin absorbant. Dans une réalisation recommandée la seconde zone suivant laquelle les plis longitudinaux supplémentaires sont libres de se déplier s'étend sur une longueur au moins égale à celle de la zone d'entrejambes de l'article d'hygiène, et de préférence au moins égale à celle de l'ouverture dans la feuille de surface.

Dans une autre réalisation recommandée les gaines fermées des premiers plis longitudinaux sont rabattues vers l'intérieur en direction de l'axe longitudinal de l'article d'hygiène et fixées à la partie sous-jacente de la feuille de surface intérieure, par exemple sur toute leur longueur ou par intermittence, de façon à former des plis en forme de Z.

Selon un autre mode de réalisation préféré les plis longitudinaux supplémentaires sont réalisés selon un pliage en forme de Z ou de S.

Selon une autre variante de réalisation l'article d'hygiène absorbant comporte en outre une feuille intermédiaire, perméable aux liquides, située entre le coussin absorbant et la feuille de surface intérieure, cette feuille intermédiaire recouvrant le coussin au moins dans la zone de l'ouverture longitudinale et étant fixée au coussin absorbant. De préférence encore, cette feuille intermédiaire, fixée au coussin, s'étend sur toute la longueur de l'article d'hygiène absorbant et a une largeur supérieure à celle de l'ouverture longitudinale. La feuille de surface intérieure peut alors être fixée à la feuille intermédiaire, à l'exception, comme précédemment d'une zone située sous l'ouverture.

La suite de la description qui concerne un mode de réalisation illustratif et non limitatif de l'invention se réfère aux figures annexées qui représentent, respectivement :
- figure 1, une vue de dessus en perspective, d'une couche-culotte selon la présente invention,
- figure 2, une vue en coupe schématique faite suivant la ligne II-II de la figure 1,
- figure 3, une vue en coupe schématique faite suivant la ligne III-III de la figure 1.

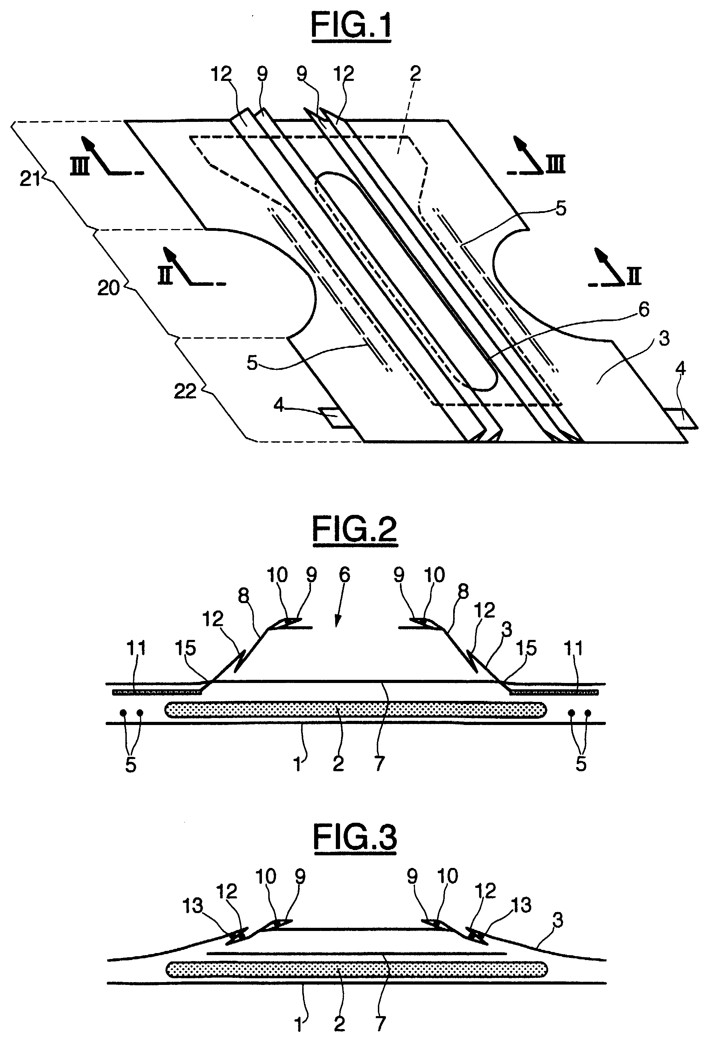

En se référant aux figures 1 à 3, la couche-culotte représentée comprend, de façon connue en soi, une feuille de support 1 imperméable aux liquides, par exemple un film de polyéthylène, un coussin absorbant 2, par exemple en pâte fluff de cellulose avec incorporation éventuelle de particules de matériau superabsorbant, et une feuille de surface intérieure 3 perméable aux liquides, par exemple en voile de non-tissé hydrophile ou traité avec des zoneshydrophobes. Les deux feuilles 1 et 3 ont les mêmes dimensions et la même forme en sablier, c'est-à-dire une forme rectangulaire avec deux échancrures latérales opposées délimitant dans le sens de la longueur de la couche-culotte, une zone d'entrejambes 20 de largeur réduite entre deux zones d'extrêmité avant 21 et arrière 22 de largeur accrue dont les bords transversaux définissent les bords transversaux avant et arrière de la couche-culotte

Le coussin absorbant 2 disposé entre les feuilles 1 et 3 présente une forme en T, mais toute autre forme pourrait être envisagée. Les deux feuilles 1 et 3 sont reliées entre-elles, par exemple par collage, sur le pourtour du coussin 2. Des moyens d'attache 4 sont prévus pour permettre la fermeture de la couche-culotte autour de la taille d'un utilisateur.

Des éléments élastiques 5 longitudinaux constitués, par exemple, chacun par un ou plusieurs brins ou fils élastiques sont fixés à l'état tendu à la feuille de support 1, au moins dans la zone d'entrejambes, entre le bord longitudinal de la feuille 1 et le bord longitudinal correspondant du coussin absorbant 2.

Comme le montre la figure 1, la feuille de surface intérieure 3 comporte une ouverture 6, généralement de forme oblongue ou allongée, symétrique par rapport à l'axe longitudinal médian de la couche-culotte, centrée au-dessus du coussin absorbant 2 et de largeur inférieure à la largeur du coussin absorbant dans la zone d'entrejambes de façon à faciliter la pénétration de l'urine et des selles dans celui-ci. De préférence, cette ouverture a une largeur inférieure à la moitié de la largeur du coussin 2 dans la zone d'entrejambes.

Bien évidemment les dimensions de cette ouverture 6 dépendent de la taille de l'utilisateur auquel est destinée la couche-culotte

Dans une réalisation donnée à titre d'exemple, pour une couche-culotte de la taille dite "Maxi" (8-18 kg) l'ouverture 6 est sensiblement rectangulaire ayant une longueur de 300 mm et une largeur de 50 mm.

De préférence, la couche-culotte comporte une bande ou feuille intermédiaire 7 perméable aux liquides, par exemple en non-tissé hydrophile, s'étendant sur toute la longueur de la coucheculotte et de largeur égale ou supérieure à la largeur du coussin absorbant 2 dans la zone d'entrejambes et donc également à la largeur de l'ouverture longitudinale 6. De plus, la longueur de cette feuille intermédiaire 7 pourrait également être inférieure à celle de la couche-culotte, et même à celle du coussin absorbant pour autant qu'elle soit supérieure à la longueur de l'ouverture 6, afin d'éviter tout contact direct entre la peau de l'utilisateur et le coussin absorbant. De préférence, cette feuille intermédiaire est fixée au coussin absorbant 2. La feuille de surface intérieure 3 est fixée d'une part à cette feuille intermédiaire 7 par des lignes de collage 15 et d'autre part au reste de la couche-culotte par des zones de collage 11, à l'exception d'une zone située en-dessous de l'ouverture longitudinale 6 et de dimension supérieure à celle-ci (Par exemple, pour les dimensions de l'ouverture longitudinale 6 données précédemment, cette zone peut être une zone rectangulaire de 360 mm de longueur sur 100 mm de large), de façon à former une poche de confinement continue au-dessus et à la périphérie du coussin absorbant, comprenant deux volets longitudinaux d'étanchéité 8 se prolongeant par deux volets transversaux, dont les bords libres sont constitués par les bords de l'ouverture 6.

La feuille de surface 3 comporte également deux plis longitudinaux parallèles 9, s'étendant sur toute la longueur de la couche-culotte et disposés de part et d'autre de l'ouverture longitudinale 6 à proximité des bords longitudinaux de celle-ci. Les bords inférieurs des plis 9 sont réunis par une ligne de fixation longitudinale, par exemple par une ligne de collage, sur toute la longueur des plis, pour former des gaines fermées. Des éléments élastiques longitudinaux 10 sont disposés à l'état tendu à l'intérieur des plis dans la partie supérieure des gaines fermées formées par ces plis 9. Ces plis peuvent, de préférence, être rabattus vers l'intérieur de la couche-culotte en direction de l'axe longitudinal médian de celle-ci, et fixés à la partie sous-jacente de la feuille de surface intérieure 3, par exemple par une ligne de collage en continu ou par intermittence, de façon à former des doubles plis en forme de Z ou de S.

La feuille de surface 3 est pourvue en outre de deux plis longitudinaux supplémentaires 12, s'étendant sur toute la longueur de la couche-culotte, et disposés symétriquement par rapport à l'ouverture 6 entre les premiers plis 9 et la zone de fixation longitudinale de la feuille de surface 3 à la feuille intermédiaire 7 recouvrant le coussin absorbant 2 et au reste de la couche-culotte.

Ces plis supplémentaires 12 peuvent être obtenus par un procédé de pliage de la feuille de surface 3 identique à celui utilisé pour former les premiers plis 9, ou par tout autre procédé, et se présenter par exemple sous la forme de Z comme le montrent les figures 1 à 3 ; toutefois d'autres formes de pliage, telles que par exemple un pli en accordéon, ou en S, peuvent être envisagées.

Comme représenté sur la figure 3, chacun de ces plis supplémentaires 12 est solidarisé de la feuille de surface 3 à ses extrémités longitudinales, à proximité des bords transversaux de la couche-culotte, par exemple par une ou plusieurs zones de collage 13 ou tout autre moyen de fixation (thermoscellage, ultrasons) de longueur réduite, par exemple de 1 à 2 cm, permettant aussi le scellage ensemble des deux faces du pli. De la sorte les extrémités des plis 12 qui pourraient former des canaux de diffusion de l'urine vers l'extérieur de la couche-culotte, se trouvent fermées, évitant ainsi des risques de fuites longitudinalement le long de ces plis.

Par contre chaque pli 12 est libre de se déplier sur la majeure partie de sa longueur, c'est-à-dire sur une longueur au moins égale à celle de la zone d'entrejambes de la couche-culotte, et de préférence au moins égale à celle de l'ouverture 6.

On peut aussi envisager la formation d'une multiplicité de plis supplémentaires répartis sur la largeur de chaque volet d'étanchéité 8.

Lorsque la couche-culotte est placée sur le corps de l'utilisateur, sous l'action des éléments élastiques longitudinaux 10 fixés dans les premiers plis 9, qui soulèvent les bords de l'ouverture 6 de la feuille de surface 3, le dépliement des plis 12 entraîne une augmentation de la hauteur des volets longitudinaux d'étanchéité 8, donc une meilleure adaptation de ceux-ci au corps de l'utilisateur, sans que la largeur de l'ouverture 6 ne soit modifiée au cours du processus de fabrication. En effet, comme la hauteur de chaque volet longitudinal d'étanchéité 8 formant la majeure partie de la poche de confinement est déterminée par la distance entre le bord longitudinal correspondant de l'ouverture 6 et la ligne longitudinale 15 de fixation de la feuille de surface 3 à la feuille intermédiaire 7, si cette ligne 15 ne peut pas être déplacée vers les bords longitudinaux de la couche-culotte pour des raisons d'étanchéité, la seule possibilité restante au niveau du procédé de fabrication est de réduire la largeur de l'ouverture 6. Or une telle réduction ne peut qu'être néfaste à une bonne acquisition et un stockage efficace des selles et de l'urine.

On a réalisé ainsi une couche-culotte qui permet une meilleure étanchéité de la poche de confinement ainsi formée vis-à-vis des fuites d'urine, tout en maintenant une excellente acquisition et retenue de l'urine et des selles dans le coussin absorbant.

## Revendications

1. Article d'hygiène absorbant jetable, tel qu'une couche-culotte, comprenant une feuille extérieure de support (1) imperméable aux liquides, de forme générale rectangulaire, un coussin absorbant (2), de forme générale rectangulaire, fixé sur ladite feuille extérieure de support, une feuille de surface intérieure (3) perméable aux liquides recouvrant le coussin absorbant, fixée sur son pourtour à la feuille extérieure de support, des moyens d'attache (4) pour permettre la fermeture de la couche-culotte autour de la taille d'un utilisateur, des éléments élastiques (5) longitudinaux fixés à l'état tendu à la feuille de support à l'extérieur des bords longitudinaux du coussin absorbant (2), au moins dans une zone d'entrejambes (20), ladite feuille de surface intérieure (3) comportant une ouverture longitudinale (6), centrée sur le coussin absorbant (2) et de largeur inférieure à celui-ci, cette feuille de surface intérieure (3) étant fixée au coussin absorbant, directement ou indirectement, sauf dans une zone située en-dessous de l'ouverture longitudinale (6) et de dimension supérieure à celle-ci, ladite feuille de surface intérieure (3) comportant également au moins deux premiers plis longitudinaux (9), sensiblement parallèles à l'axe longitudinal médian de la couche-culotte, s'étendant sur tout la longueur de ladite couche-culotte de part et d'autre de l'ouverture longitudinale (6), chacun desdits plis (9) formant une gaine fermée dans laquelle est fixé, à l'état tendu, au moins un élément élastique longitudinal (10), caractérisé en ce que : ladite feuille de surface intérieure (3) est pourvue d'au moins deux plis longitudinaux supplémentaires (12), s'étendant sur toute la longueur de la couche-culotte, et disposés respectivement entre chacun des premiers plis longitudinaux (9) et la zone longitudinale latérale de fixation (11) correspondante de la feuille de surface (3) au reste de la couche-culotte.

2. Article selon la revendication 1, caractérisé en ce que chacun des plis longitudinaux supplémentaires (12) est solidarisé de la feuille de surface intérieure (3) à chacune de ses extrémités longitudinales, à proximité des bords transversaux de la couche-culotte, suivant une première zone de longueur réduite, et se trouve en configuration libre pour son dépliement suivant une seconde zone correspondant à la majeure partie de la longueur de la couche-culotte.

3. Article selon la revendication 2, caractérisé en ce que la seconde zone suivant laquelle les plis longitudinaux supplémentaires (12) sont libres de se déplier s'étend sur une longueur au moins égale à celle de la zone d'entrejambes (20) de la couche-culotte, et de préférence au moins égale à celle de l'ouverture longitudinale (6) dans la feuille de surface (3).

4. Article selon l'une quelconque des revendications 1 à 3, caractérisé en ce que les gaines fermées des premiers plis longitudinaux (9) sont rabattues vers l'intérieur en direction de l'axe longitudinal de la couche-culotte et fixées à la partie sous-jacente de la feuille de surface intérieure (3), de façon à former des plis en forme de Z.

5. Article selon l'une quelconque des revendications 1 à 3, caractérisé en ce que les plis longitudinaux supplémentaires (12) sont réalisés selon un pliage en forme de Z ou de S.

6. Article selon l'une quelconque des revendications 1 à 5, caractérisé en ce qu'il comprend en outre une feuille intermédiaire (7), perméable aux liquides, située entre le coussin absorbant (2) et la feuille de surface intérieure (3), cette feuille intermédiaire recouvrant le coussin absorbant au moins dans la zone de l'ouverture longitudinale (6) et lui étant fixée.

7. Article selon la revendication 6, caractérisé en ce que ladite feuille intermédiaire (7) s'étend sur toute la longueur de la couche-culotte et a une largeur supérieure à celle de l'ouverture longitudinale (6) et que la feuille de surface intérieure (3) est fixée à celle-ci, à l'exception de ladite zone située en dessous de l'ouverture (6).

## Patentansprüche

1. Wegwerfbarer absorbierender Hygieneartikel, wie z.B. eine Hosenwindel, mit einem flüssigkeitsundurchlässigen äußeren Stützblatt (1) von im wesentlichen rechteckiger Form, einem auf dem äußeren Stützblatt angebrachten, absorbierenden Kissen (2) von im wesentlichen rechteckiger Form, einem das absorbierende Kissen abdeckenden, flüssigkeitsdurchlässigen inneren Deckblatt (3), welches an seinem Rand an dem äußeren Stützblatt befestigt ist, Befestigungsmitteln (4), die das Schließen der Hosenwindel um die Taille eines Benutzers ermöglichen, langgestreckten elastischen Elementen (5), die in gespanntem Zustand zumindest in einer Zwickelzone (20) außerhalb der Längsränder des absorbierenden Kissens (2) an dem Stützblatt befestigt sind, wobei das innere Deckblatt (3) in der Mitte des absorbierenden Kissens (2) eine langgestreckte Öffnung (6) von einer Breite geringer als der Breite desselben aufweist, das innere Deckblatt (3) außer in einer Zone unterhalb der langgestreckten Öffnung (6) direkt oder indirekt an dem absorbierenden Kissen befestigt ist und größer ist als dieses, das innere Deckblatt (3) ebenso mindestens zwei erste langgestreckte Falten (9), im wesentlichen parallel zu der Mittellängsachse der Hosenwindel aufweist, die sich über die gesamte Länge der Hosenwindel beiderseits der langgestreckten Öffnung (6) erstrecken, und jede der Falten (9) eine geschlossene Hülle bildet, in welcher mindestens ein langgestrecktes elastisches Element (10) im gespannten Zustand befestigt ist, dadurch gekennzeichnet, daß das innere Deckblatt (3) mindestens zwei zusätzliche langgestreckte Falten (12) aufweist, die sich über die gesamte Länge der Hosenwindel erstrecken und jeweils zwischen jeweils einer der erstgenannten langgestreckten Falten (9) und der langgestreckten seitlichen Befestigungszone (11) entsprechend dem Deckblatt (3) auf dem Rest der Hosenwindel angeordnet sind.

2. Artikel nach Anspruch 1, dadurch gekennzeichnet, daß eine jede der zusätzlichen langgestreckten Falten (12) an ihren beiden Längsrändern in der Nähe der querverlaufenden Ränder der Hosenwindel in einer ersten Zone geringerer Länge mit dem inneren Deckblatt (3) verbunden ist und zu ihrer Entfaltung in einer zweiten Zone entsprechend dem größeren Teil der Länge der Hosenwindel freiliegt.

3. Artikel nach Anspruch 2, dadurch gekennzeichnet, daß die zweite Zone, in welcher die zusätzlichen langgestreckten Falten (12) zu ihrer Entfaltung freiliegen, sich über eine Länge von mindestens derjenigen der Zwickelzone (20) der Hosenwindel und vorzugsweise mindestens derjenigen der langgestreckten Öffnung (6) in dem Deckblatt (3) erstreckt.

4. Artikel nach irgendeinem der Ansprüche 1 bis 3, dadurch ***gekennzeichnet,*** daß die geschlossenen Hüllen der ersten langgestreckten Falten (9) nach innen zu in Richtung zur Längsachse der Hosenwindel hin umgeschlagen und an der darunterliegenden Partie des inneren Deckblatts (3) befestigt sind in einer Weise, um Z-förmige Falten zu bilden.

5. Artikel nach irgendeinem der Ansprüche 1 bis 3, dadurch gekennzeichnet, daß die zusätzlichen langgestreckten Falten (12) mit Z- oder S-förmiger Faltung ausgeführt sind.

6. Artikel nach irgendeinem der Ansprüche 1 bis 5, dadurch gekennzeichnet, daß er des weiteren zwischen dem absorbierenden Kissen (2) und dem inneren Deckblatt (3) ein flüssigkeitsdurchlässiges Zwischenblatt (7) aufweist, welches das absorbierende Kissen zumindest innerhalb der Zone der langgestreckten Öffnung (6) abdeckt und daran befestigt ist.

7. Artikel nach Anspruch 6, dadurch gekennzeichnet, daß das Zwischenblatt (7) sich über die gesamte Länge der Hosenwindel erstreckt und eine Breite größer als diejenige der langgestreckten Öffnung (6) hat und daß das innere Deckblatt (3) daran befestigt ist mit Ausnahme der genannten Zone unterhalb der Öffnung (6).

## Claims

1. Disposable absorbent sanitary article, such as a diaper, comprising a liquid-tight outer backing sheet (1) of generally rectangular shape, an absorbent pad (2) of generally rectangular shape fixed on said outer backing sheet, a liquid-pervious inner surface sheet (3) covering the absorbent pad, fixed over its periphery to the outer backing sheet, fastening means (4) to enable the diaper to be closed around a user's waist, longitudinal resilient elements (5) fixed in the stretched state to the backing sheet outside the longitudinal edges of the absorbent pad (2), at least in a crotch region (20), said inner surface sheet (3) including a longitudinal opening (6) which is centered on the absorbent pad (2) and narrower than the latter, this inner surface sheet (3) being directly or indirectly fixed to the absorbent pad, except in a region which is located below the longitudinal opening (6) and which is larger than it, said inner surface sheet (3) also including at least two first longitudinal folds (9) which are substantially parallel to the median longitudinal axis of the diaper, extending over the whole length of this diaper on either side of the longitudinal opening (6), each of said folds (9) forming a closed sleeve in which at least one longitudinal resilient element (10) is fixed, in the stretched state, characterized in that: said inner surface sheet (3) has at least two additional longitudinal folds (12) extending over the whole length of the diaper and arranged respectively between each of the first longitudinal folds (9) and the corresponding lateral longitudinal region (11) in which the surface sheet (3) is fixed to the rest of the diaper.

2. Article according to Claim 1, characterized in that each of the additional longitudinal folds (12) is secured to the inner surface sheet (3) at each of its longitudinal ends, close to the transverse edges of the diaper, in a first, shorter region and is configured so as to be free to unfold in a second region corresponding to the greater part of the length of the diaper.

3. Article according to Claim 2, characterized in that the second region in which the additional longitudinal folds (12) are free to unfold extends over a length which is at least equal to that of the crotch region (20) of the diaper and preferably at least equal to that of the longitudinal opening (6) in the surface sheet (3).

4. Article according to any one of Claims 1 to 3, characterized in that the closed sleeves of the first longitudinal folds (9) are folded inward in the direction of the longitudinal axis of the diaper and fixed to the part lying under the inner surface sheet (3) so as to form Z-shaped folds.

5. Article according to any one of Claims 1 to 3, characterized in that the additional longitudinal folds (12) are produced by means of Z- or S-shaped folding.

6. Article according to any one of Claims 1 to 5, characterized in that it also comprises a liquid-pervious intermediate sheet (7) located between the absorbent pad (2) and the inner surface sheet (3), this intermediate sheet covering the absorbent pad at least in the region of the longitudinal opening (6) and being fixed to it.

7. Article according to Claim 6, characterized in that said intermediate sheet (7) extends over the whole length of the diaper and is wider than the longitudinal opening (6) and in that the inner surface sheet (3) is fixed to it, except for said region located below the opening (6).
